(19) Europäisches Patentamt
European Patent Office
Office européen des brevets

(11) Numéro de publication: 0 058 286
A1

(12) **DEMANDE DE BREVET EUROPEEN**

(21) Numéro de dépôt: 81810042.2

(22) Date de dépôt: 12.02.81

(51) Int. Cl.³: C 07 D 233/64
C 07 D 203/12, C 07 C 129/08
C 07 C 149/437

(43) Date de publication de la demande:
25.08.82 Bulletin 82/34

(84) Etats contractants désignés:
AT BE DE FR GB IT NL SE

(71) Demandeur: Société de Recherches et de Synthèses Organiques SA
12 rue Marziano
CH-1227 Genève(CH)

(72) Inventeur: Baudet, Pierre
15 chemin de Passoret 1227
Genève(CH)

(54) **Isomères géométriques de cyano-guanidines et leurs procédés de préparation.**

(57) Cyano-guanidines de formule générale:

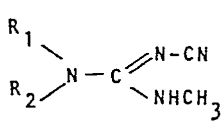

(forme tautomère: amino méthyle et imino-nitrile)

dans laquelle

(a) $R_1$=H ; $R_2$= $-CH_2CH_2SH$

(b) $R_1$=H ; $R_2$= 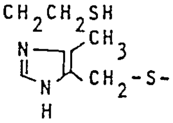

(c) 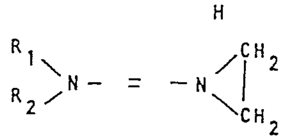

et plusieurs de leur préparation respective.

EP 0 058 286 A1

# Isomères géométriques de cyano-guanidines et leurs procédés de préparation

## DESCRIPTION

Dans le préambule il est nécessaire de définir le phénomène de l'isomérie géométrique afin d'expliquer la portée de l'invention et dans la suite de la description de présenter le caractère nouveau des composés de l'invention et de leurs procédés de préparation.

L'isomérie géométrique est le phénomène de la chimie organique caractérisé par l'asymétrie provenant d'au moins deux substituants différents, liés à deux atomes engagés l'un avec l'autre par une double liaison, par ex. la double liaison carbone-carbone ,

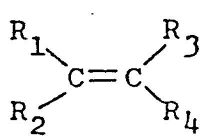

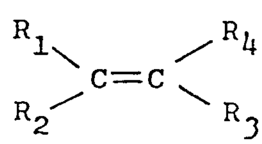

ou la double liaison carbone-azote,

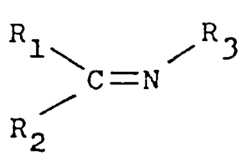

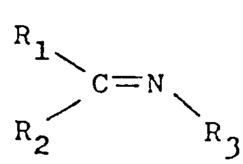

Les isomères géométriques déterminés par la double liaison

carbone-carbone et les substituants $R_1$, $R_2$, $R_3$, $R_4$ sont appelés cis et trans. Les isomères géométriques déterminés par la double liaison carbone-azote et les substituants $R_1$, $R_2$, $R_3$ sont appelés syn et anti. Les isomères géométriques peuvent exister en pairs dans le rapport 1/1 ou dans un rapport différent selon l'énergie nécessaire à leur formation, selon leur stabilité. Un seul peut exister. Le passage d'un isomère géométrique à l'autre est possible si le double liaison disparaît temporairement, permettant la rotation d'un atome de carbone ou d'azote autour de l'axe de liaison $Sp^2$, soit en passant par un intermédiaire, par ex. un tautomère, soit seulement par un état de transition (1) selon un "bond-rotational mechanism" ou (2) un "lateral shift mechanism",

(1)

(2)

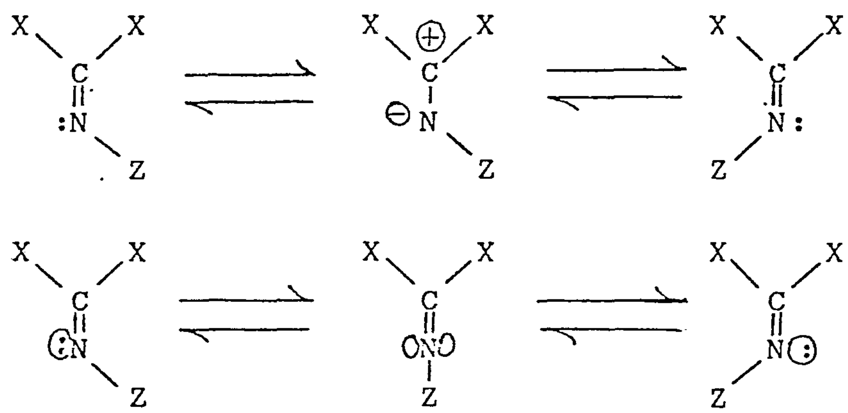

Les inversions de configurations, liées à un état d'équilibre et fournissant les deux isomères géométriques stables, sont relativement rares. Pour parvenir à préparer les deux isomères géométriques stables, il est souvent nécessaire de créer un réactif qui possède déjà la forme isomère géométrique souhaitée et de l'engager - pour obtenir le composé final - avec un autre réactif. Ainsi, l'existence d'un isomère géométrique, souvent, ne dépend pas de l'existence de l'autre.

La préparation et l'isolement des deux isomères géométriques d'une guanidine n'ont pas encore, à notre connaissance, été réalisés ; il n'en va pas de même des

0058286

amidines ("Investigation of Geometrical isomerism and tautomerism in some new Benzothiazol-2-carboxamidines" Thèse n° 1680, 1975, Université de Genève).

C'est ainsi que nous avons trouvé que des guanidines déjà connues sous une forme géométrique pouvaient exister dans l'autre forme isomère géométrique, il s'agit de la N-(2-mercapto-éthyl)-N'-méthyl-N''-cyano-guanidine et de la N-méthyl-N'-cyano-N''- [2-((4-méthyl-5-imidazolyl)-méthylthio)-éthyl] -guanidine. Ces deux guanidines possèdent la forme tautomère : amino-méthyle et imino-nitrile.

Dans le cas particulier des composés de l'invention, nous devons considérer les propriétés qui leur sont propres, c-à-d celles des N-cyano-guanidines.

Les N-cyano-guanidines tautomère amino-méthyle et imino-nitrile, isomère Syn($-NHCH_3$),

I

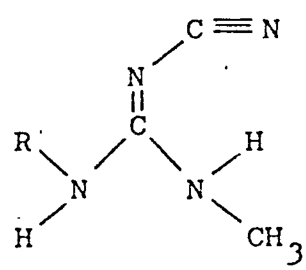

n'existaient pas encore. Cela provient du fait que les synthèses jusqu'à présent connues des N-cyano-guanidines ne fournissaient que les isomères anti($-NHCH_3$) de la formule :

II

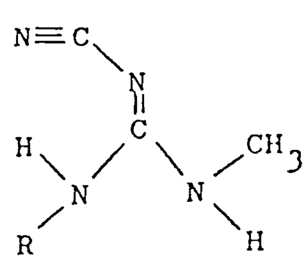

La cyano-guanidine de la formule I et la cyano-guanidine de la formule II (R identique), en solution, ne sont pas en équilibre l'une avec l'autre. Cela revient à dire qu'à partir de la N-cyano-guanidine de la formule I on ne peut pas obtenir la N-cyano-guanidine de la formule II et vice versa. Une barrière d'énergie empêche donc l'établissement de l'équilibre. Cette barrière réside dans la rapide inversion du groupe $-N-C{\equiv}N$ et des plus lentes rotations des groupes $-NHCH_3$ et -NHR.

On sait en effet (H. Kessler Angew. Chem. 82, n° 6, 237, 1970) que les imines, les oximes, les hydrazones, les guanidines s'isomérisent en milieu dissout :

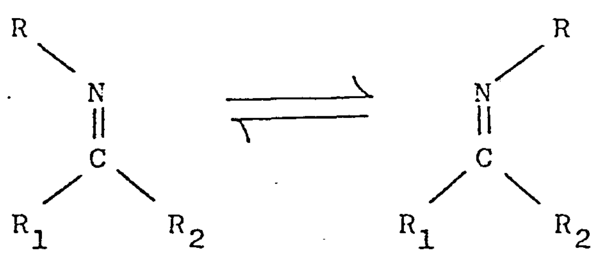

La vitesse de l'isomérisation dépend de la nature de $R_1$, $R_2$ et R. Si $R = -C{\equiv}N$, la vitesse de l'isomérisation est grande et si en plus $R_1$ et $R_2$ sont $-NHCH_3$, elle le sera encore davantage. Cela provient du fait que l'état de transition de l'isomérisation de cette guanidine requiert peu d'énergie.

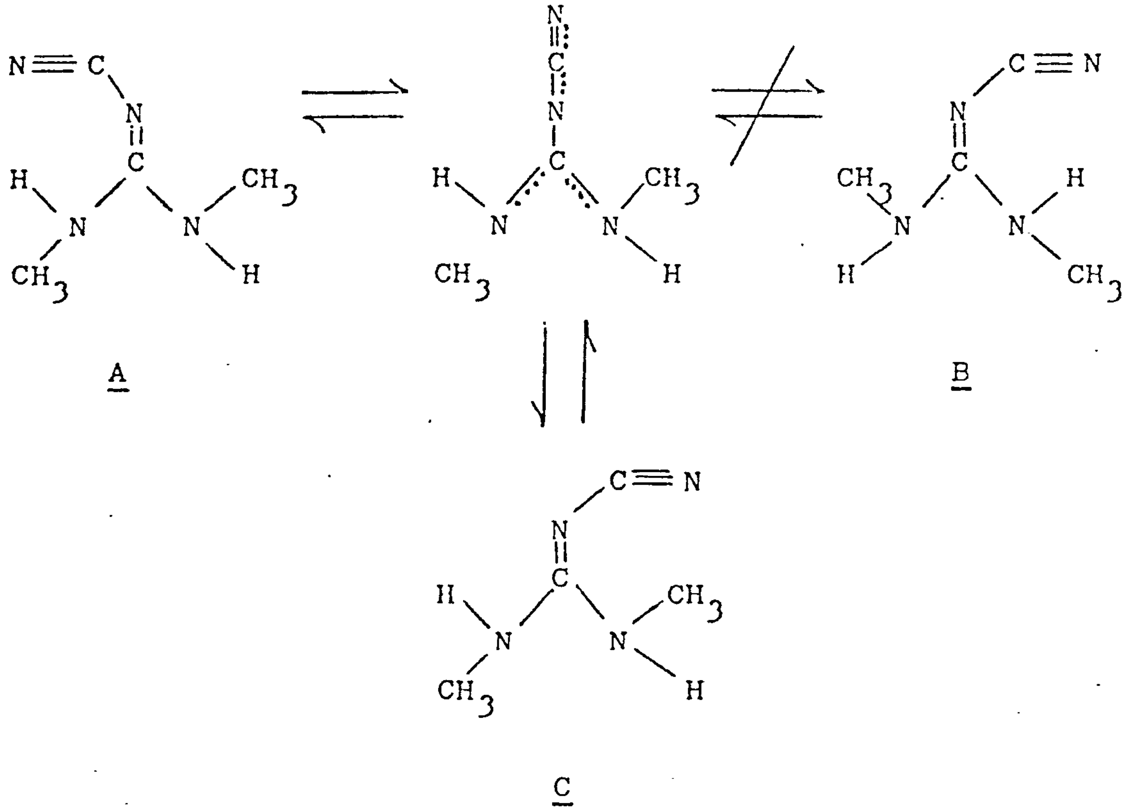

A B

C

On remarque que A ne fournit pas l'isomère B mais l'isomère instable C (qui n'existe qu'en solution). Cela provient de la barrière d'énergie évoquée plus haut et que détermine la vitesse plus grande de l'inversion de $=N-C\equiv N$ par rapport à la vitesse plus petite des rotations de $-NHCH_3$

Ce phénomène est bien mis en évidence par C.G. MacCarty et D.M. Wieland (Tetrahedron letters 22, 1787, 1969) dans leur étude de résonance magnétique nucléaire dynamique de la N-cyano-N',N''-méthyl-guanidine deutérée, pour laquelle ils déterminent : $T_c$ = $-45^oc$. $\Delta \overset{\ddagger}{G_c}$ (Kcal/mole) = 12,3.

L'isomérisation décrite ne résulte pas de l'inversion de $=N-C\equiv N$ simultanément avec les rotations des deux groupes $NDCH_3$, car alors, à l'équilibre, les molécules obtenues seraient identiques et la non équivalence magnétique des

fonctions $-CH_3$ serait observée quelque soit la température.

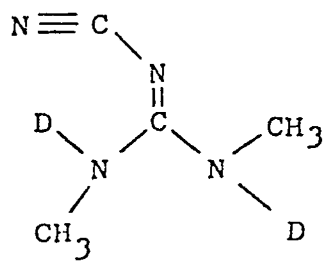

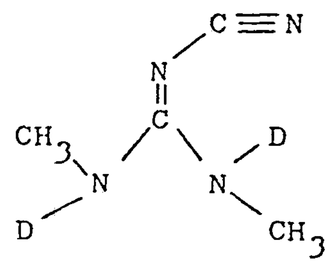

En réalité, l'inversion de $-N-C\equiv N$ est plus rapide que les rotations de $-NDCH_3$, et ainsi l'isomérisation fournit un isomère géométrique, instable, non isolable :

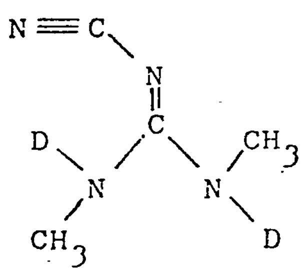

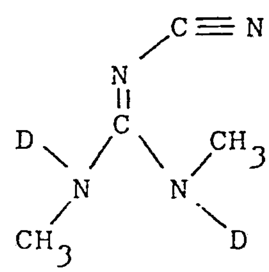

Le mécanism de ces isomérisations s'explique mieux par "rotational-bond mechanism" que par un "lateral shift mechanism" ; en effet, le groupe $>C=N-C\equiv N:$ délocalise facilement deux électrons selon $>C=N-C\equiv N \longleftrightarrow >C-N=C=\bar{N}:$ ou dans un état de transition seulement $>C \cdots N \cdots C \cdots N:$ . On sait par ailleurs que l'anion $-\overset{\ominus}{N}-C\equiv N$ peut être stabilisé par un hétéroatome voisin, comme par ex. le soufre, $R-\overset{+}{S}-\bar{N}-C\equiv N:$ .

L'isomérisation des N-cyano-guanidines ne peut avoir lieu par le déplacement temporaire de la double liaison - créant un tautomère - car la barrière d'énergie décrite plus haut s'oppose également à l'équilibre tautomérique.

Ainsi, les composés de l'invention la N-méthyl-N'-cyano-N''(2-mercapto-éthyl)-guanidine et la N-méthyl-N'-cyano-N''- [2-((4-méthyl-5-imidazolyl)-méthylthio)-éthyl] -guanidine, tautomère : amino-méthyle et imino-nitrile,

isomère géométrique Syn($-NHCH_3$) de la formule :

III

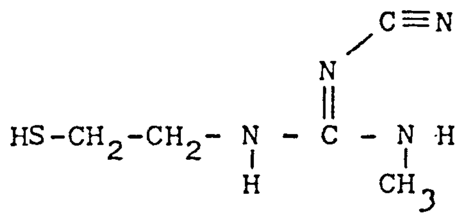

et de la formule :

IV

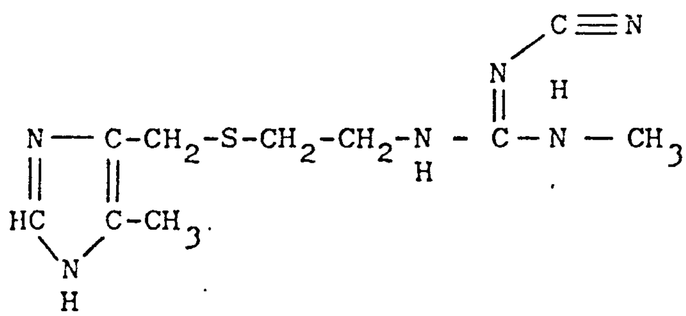

ne proviennent pas des composés de la même nomenclature, de même forme tautomère et d'isomérie anti($-NHCH_3$) (c-à-d pour le composé de la formule IV, de la cimétidine), à la suite d'un état d'équilibre ou de toutes autres transformations.

La conformation anti($-NHCH_3$) de la cimétidine a été déterminée par analyse aux rayons X (Chem. Berichte 111, 3222, 1978). Il résulte de cette analyse que l'isomère géométrique différent - le composé de l'invention dela formule IV - possède la configuration Syn($-NHCH_3$). Ainsi, en solution le composé de l'invention de la formule III et de la formule IV sont en équilibre avec un conformère instable, non isolable, à la suite de la rapide inversion de $-N-C\equiv N$

et de la lente rotation de $-NHCH_3$ et -NHR :

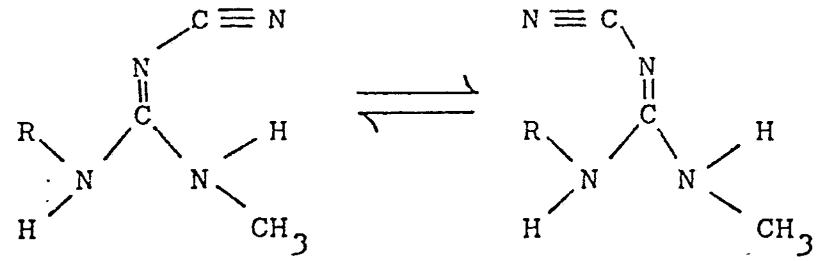

stable instable

R= $HS-CH_2-CH_2-$ = 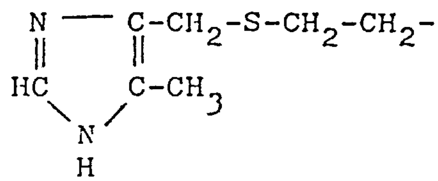

Les composés de l'invention doivent donc être synthétisé à partir d'un réactif intermédiaire qui possède déjà l'isomérie Syn($-NHCH_3$) voulue. Il s'agit de la N-(N'méthyl-N''-cyano-amidino)-aziridine, tautomère amino-méthyle et imino-nitrile, isomère géométrique Syn($-NHCH_3$) de la formule :

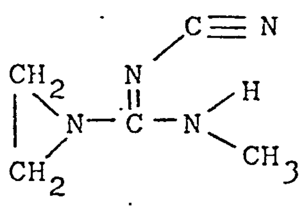

L'aziridine de la formule V additionne la fonction -SH du 4-méthyl-5-mercaptométhyl-imidazole fournissant le composé de l'invention de la formule IV. L'aziridine de la formule V additionne le terbutylxanthogénate de potassium ou de sodium fournissant le composé de la formule III de l'invention.

La synthèse de la N-(N'-méthyl-N''-cyano-amidino)-aziridine (tautomère : amino-méthyle et imino-nitrile, isomère

Syn($-NHCH_3$) est réalisé stéréospécifiquement par l'action de dérivés mercurique de la cyanamide ou argento de la cyanamide sur la N-(N-méthyl-thiono)-aziridine dans laquelle la fonction thio-carbonyle est remplacée par la fonction imino-nitrile.

Le composé de l'invention de la formule IV peut être également préparé à partir de la N-méthyl-N'-cyano-N''-(2-mercapto-éthyl)-guanidine (tautomère : amino-méthyle et imino-nitrile et isomère géométrique Syn($-NHCH_3$) (formule III) réagissant sur le 4-méthyl-5-chloro-méthyl-imidazole.

La cristallisation du composé de l'invention de la formule IV (isomère Syn($-NHCH_3$) peut être effectuée par ex. dans de l'eau, l'acétonitrile, l'isopropanol, le mélange éthanol-éther.

Il est intéressant d'observer que la N-(N'-méthyl-thiono)-aziridine et que la N-(N'-méthyl-N''-cyano-amidino)-aziridine (V) ont un caractère basique. Cela provient probablement de la géométrie pyramidale de l'azote des restes aziridine.

Les guanidines peuvent donner des colorations avec le nitroprussiate de sodium (voir J. P. Greenstein, M. Winitz, Chemistry of amino-acids, J. Wiley and Sons, New York - London Vol. 3, p. 1842) ; l'aziridine de la formule V se colore en bleu .

Les isomères géométriques Syn($-NHCH_3$) et Anti($-NHCH_3$) de la N-méthyl-N'-cyano-N''- [2-((4-méthyl-5-imidazolyl)-méthylthio)-ethyl ]-guanidine se distinguent par leur point de fusion, par leur mobilité en chromatographie en couche mince ($R_f$: 0,34 Syn($-NHCH_3$) ; $R_f$ = 0,29 Anti ($-NHCH_3$) n-butanol-ac, acétique-eau 10/2/5 v.par exemple.

Déposées sur une plaque de silica gel 60 F 254 ou après chromatographie sur cette même silice, l'isomère Syn

($-NHCH_3$) donne une coloration bleu-ciel et l'isomère Anti ($-NHCH_3$) une coloration rose avec le réactif au nitroprussiate.

Le composé de l'invention caractérisé par la formule IV isomère géométrique Syn($-NHCH_3$) et les sels de ce composé thérapeutiquement convenables, ainsi que les diverses formes cristallines polymorphes qu'il peut présenter, antagonisent l'activité de l'histamine dans les récepteurs appelés $H_2$, par lesquels l'histamine stimule la production de l'acide chlorhydrique gastrique par les cellules pariétales.

Le composé de l'invention de la formule IV, isomère Syn ($-NHCH_3$) ainsi que ses sels thérapeutiquement convenables peuvent être utilisés dans le traitement de l'ulcère gastrique et duodénale. Le composé de l'invention de la formule IV, isomère Syn($-NHCH_3$) présente sur l'isomère Anti ($-NHCH_3$) de la même formule l'avantage d'une biodisponibilité meilleure à la suite d'une meilleure solubilité des cristaux, probablement à la suite de liaisons hydrogène intra-moléculaires différentes.

Les excipients convenables pour la mise en oeuvre pharmaceutique sont par ex. le lactose, le saccharose, le talc, la gélatine, la gomme arabique, l'huile d'olive.

Exemple 1

N-aziridino-N'-méthyl-N''-cyano-guanidine (tautomère: amino-méthyle et imino-nitrile, isomère géométrique à caractère basique)

A une solution de 3,8 g de 1-(N-méthyl-thiocarbamyl)-aziridine (F.46°) dans de l'acétonitrile refroidi dans de la glace, on ajoute 7,4 g d'une cyanamide mercurique et sous

agitation laisse la réaction durant les $10^{\text{ères}}$ heures dans de la glace, puis jusqu'à la $28^{\text{ème}}$ heure à température ordinaire. On filtre l'insoluble(poudre jaune, neutre, IR (nujol): 2030, 1930 $cm^{-1}$). Le filtrat est gardé à basse température, ce qui provoque la précipitation d'un amorphe jaune. On filtre, élimine le solvant sous un vide de 0,05 T à une température ne dépassant 40°C. On obtient une huile à caractère basique évident.

(1) se polymérise partiellement dans l'acide acétique en présence d'acide perchlorique (2) $R_f$: 0,75 silice GF (chloroforme-méthanol 9/1 vol. (3) coloration bleue claire après dépôt sur plaque de silice avec le réactif au nitroprussiate de sodium (4) titration avec $S_2O_3Na_2$/NaOH (Ann. Chem. 27, 540, 1955) : 98,2 % (5) IR(film) : 3220, 3040, 2980, 2900, 2160, 1600, 1545, 1440, 1410, 1360, 1375, 1190, 1150, 1070, 1010, 930, 880, 850, 810-805 $cm^{-1}$.

Exemple 2

Cyanamide mercurique

(composé A)

A une solution de 31,86 g d'acétate mercurique dans 80 ml d'eau, on ajoute 4,2 g de cyanamide dans 10 ml d'eau. On laisse décanter la suspension formée, puis centrifuge la suspension ainsi concentrée, lave avec de l'eau et sèche sous 0,5 T.

Poudre blanche très peu soluble dans les solvants organiques et l'eau ; teneur en Hg : 95,8 % de la théorie ; IR (nujol) : 2080, 2030 et 1930 $cm^{-1}$.

(composé B)

A une suspension de 27,1 g de chlorure mercurique dans 250 ml d'eau, on ajoute 9 g de cyanamide et 15,8 g de

bicarbonate d'ammonium. Après trois heures d'agitation, on filtre, lave avec de l'eau et sèche le produit sous 0,5 T.

Poudre blanche, très peu soluble dans l'eau et les solvants organiques ; teneur en Hg: 96,3 % : IR (nujol) : 2100, 1210 $cm^{-1}$.

Exemple 3

N-méthyl-N'-cyano-N''- [2((4-méthyl-5-imidazolyl)-méthylthio)-éthyl)] -guanidine (tautomère : amino-méthyle et imino-nitrile, isomère géométrique Syn($-NHCH_3$)

A une solution de 16,45 g du chlorhydrate du 4-méthyl-5-mercapto-méthyl-imidazole dans 110 ml de NaOH 1N, sous un courant d'azote ou d'argon, on ajoute une solution de 12,6 g de N-aziridino-N'-méthyl-N''-cyano-guanidine (tautomère : amino-méthyle et imino-nitrile et isomère géométrique à caractère basique). La condensation est effectuée à température ordinaire, dans une atmosphère inerte durant 15 heures. On centrifuge une petite fraction insoluble, évapore le solvant sous 0,02 T et reprend le résidu par de l'acétonitrile, élimine le NaCl et cristallise le produit par ex. à $-5^oC$.

$R_f$: 0,34 (silice GF, n-butanol-ac.acétique- eau 10/2/5 vol) ; coloration bleu-ciel après dépôt sur plaque de silice GF avec le réactif au nitroprussiate de sodium ; IR (nujol): 3200, 3070, 2160, 1620, 1580, 1518, 1690, 1420, 1340, 1305, 1280, 1260, 1235, 1220, 1200, 1155, 1070, 950, 845, 775, 700, 670, 630 $cm^{-1}$, F. 120-122° C.

$C_{10}H_{16}N_6S$(252) Calc. C 47, 61, H 6,35, N 33, 33, S 12,64 %
Tr. C 47, 53, H 6,51, N 33, 37, S 12,58 %

Exemple 4

N-méthyl-N'-cyano-N''-(2-mercapto-éthyl)-guanidine (tautomère : amino-méthyle et imino-nitrile, isomère géométrique Syn($-NHCH_3$)

A une solution dans de l'eau de 12,4 g de N-aziridino-N'-méthyl-N''-cyano-guanidine (tautomère : amino-méthyle et imino-nitrile, isomère géométrique à caractère basique),

on ajoute 15,8 g de terbutylxanthogénate de potassium. Après 8 heures de réaction à température ordinaire, on neutralise, élimine le solvant et reprend le résidu cristallin par une solution de HCl dans de l'éthanol ; après 3 heures, à température ordinaire, on isole le produit comme chlorhydrate.

Titration au méthoxyde de sodium : 99,4 % ; coloration rouge après "spray" du réactif nitroprussiate, coloration qui se manifeste immédiatement.

Exemple 5

N-méthyl-N'-cyano-N''- [2-((4-méthyl-5-imidazolyl)-méthylthio)-ethyl) ] - guanidine (tautomère : amino-éthyle et imino-nitrile, isomère géométrique Syn(-$NHCH_3$)

A une solution de 2,66 g de N-méthyl-N'-cyano-N''-(2-mercapto-éthyl)-guanidine et 2,77 g de chlorhydrate de 4-méthyl-5-chloro-méthyl-imidazole, dans de l'eau, on ajoute sous courant d'azote, 1,85 g de KOH et porte la température de réaction à 50°C et maintient le pH à 11. Après 5 heures, on élimine le solvant sous 0,02 T, reprend le résidu par de l'acétonitrile, élimine le KCl et cristallise le produit à -10°C ; F.120-122°C ; analyses comme dans l'exemple 3.

Exemple 6

Chlorhydrate de N-méthyl-N'-cyano-N''- [2((4-méthyl-5-imidazolyl)-méthylthio)-éthyl ] -guanidine (comme tautomère : amino-méthyle et imino-nitrile, isomère géométrique Syn(-$NHCH_3$)

A une solution de 12,4 g de N-aziridino-N'-méthyl-N''-cyano-guanidine dans une atmosphère d'azote, on ajoute 16,4 g de chlorhydrate de 4-méthyl-5-mercapto-méthyl-imidazole. Après 17 heures, à température ordinaire, on évapore le solvant sous 0,02 T et cristallise le chlorhydrate dans de l'éthanol par de l'éther.

Isomères géométriques de cyano-guanidines

et leurs procédés de préparation

REVENDICATIONS

(1) Cyano-guanidines de formule générale:

I 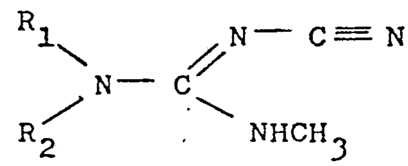

Caractérisée par la forme tautomère : amino-méthyle et imino-nitrile et par l'isomérie géométrique dans laquelle l'isomère est différent de celui des cyano-guanidines déjà connues de cette formule générale et de cette forme tautomère, et :

- dans laquelle $R_1$ est un atome d'hydrogène, $R_2$ est le reste 2-mercapto-éthyle ;
- dans laquelle $R_1$ est un atome d'hydrogène, $R_2$ est le reste 2-((4-méthyl-5-imidazolyl)-méthylthio)-éthyle ;
- dans laquelle $R_1 = R_2$ est le reste $-CH_2-CH_2$ et dont les extrêmités sont liées à l'atome d'azote (reste aziridine).

(2) Procédé selon la revendication 1 pour la préparation du composé de la formule :

II 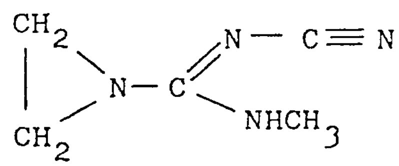

tautomère : amino-méthyle et imino-nitrile, isomère géométrique qui détermine le caractère basique du composé.

Caractérisé par ce que l'on fait réagir la 1-(N-méthyl-thio-carbamyl)-aziridine avec une cyanamide mercurique, dans un solvant convenable, comme par ex. l'acétonitrile, à une température qui ne dépasse pas la température de dimérisation et de polymérisation de l'aziridine réagissante.

(3) Procédé selon la revendication 1 pour la préparation du composé de la formule :

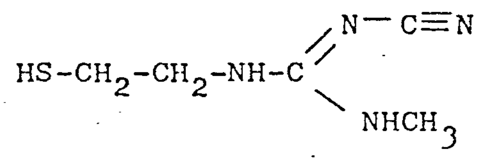

tautomère : amino-méthyle et imino-nitrile, isomère géométrique Syn($-NHCH_3$) - (différent de celui, Anti($-NHCH_3$) qui caractérise la cyano-guanidine de la même formule, déjà connue

Caractérisé par ce que l'on fait réagir la N-aziridino-N'-méthyl-N''-cyano-guanidine, tautomère : amino-méthyle et imino-nitrile, isomère géométrique qui détermine un caractère basique, dans un solvant convenable comme un alcool, l'eau, avec le terbutyl-xanthogénate de potassium ou de sodium et caractérisé par l'acidolyse du xanthate obtenu et subséquement par la neutralisation du chlorhydrate obtenu.

(4) Procédé selon la revendication 1 pour la préparation du composé de la formule :

IV 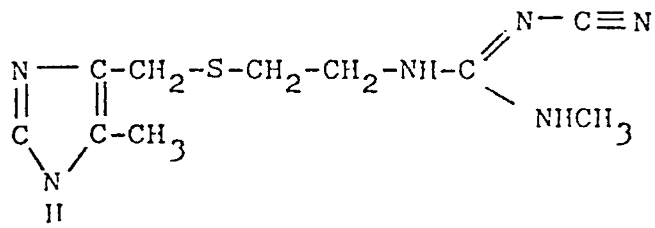

tautomère : amino-méthyle et imino-nitrile, isomère géométrique Syn($-NHCH_3$) - (différent de celui, Anti($-NHCH_3$) qui caractérise la cyano-guanidine de la même formule, déjà connue).

Caractérisé par ce que l'on fait réagir la N-aziridino-N'-méthyl-N''-cyano-guanidine, tautomère amino-méthyle et imino-nitrile, isomère géométrique qui détermine un caractère basique, avec le 4-méthyl-5-mercapto-méthyl-imidazole comme chlorhydrate dans un solvant convenable comme par ex. de l'eau en présence d'un agent alcalin convenable comme par ex. KOH ou NaOH en quantité stoechiométrique à celle du chlorhydrate, plus une quantité supplémentaire allant de très faible au point de vue de la stoechimétrie à par ex. 1/20, 1/10, 1/5, 1/1 par rapport à la quantité molaire de l'imidazole engagé.

La réaction est conduite dès le début dans une atmosphère inerte comme par ex. dans de l'azote ou de l'argon. La température de la réaction peut être une température allant de l'ordinaire jusqu'à 100$^{o}$C.

(5) Procédé selon la revendication 1 pour la préparation du composé de la formule :

V 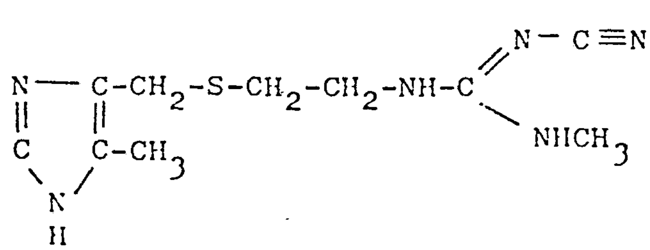 . HCl

tautomère : amino-méthyle et imino-nitrile, isomère géométrique Syn($-NHCH_3$) - (différent de celui, Anti($-NHCH_3$) qui caractérise la cyano-guanidine de la même formule, déjà connue).

Caractérisé par ce que l'on fait réagir le chlorhydrate du 4-méthyl-5-mercapto-méthyl-imidazole avec la N-aziridino-N'-méthyl-N''-cyano-guanidine (tautomère : amino-méthyle et imino-nitrile, isomère géométrique qui détermine une basicité) dans un solvant convenable comme de l'eau, un alcool, un solvant chloré, dans une atmosphère inerte comme par ex. dans de l'azote ou de l'argon. La température peut aller par ex. de la température ordinaire jusqu'à 100°C.

(6) Procédé selon la revendication 1 pour la préparation du composé de la formule :

VI 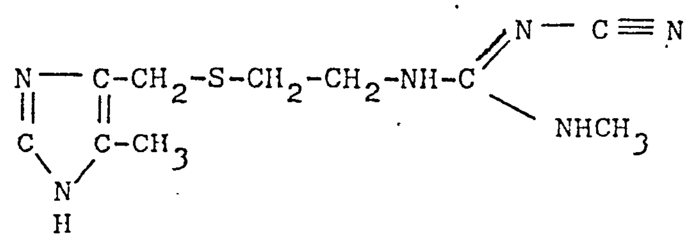 . HCl

tautomère : amino-méthyle et imino-nitrile, isomère géométrique Syn($-NHCH_3$) -(différent de celui, Anti($-NHCH_3$) du composé d'une éventuelle même formule).

Caractérisé par ce que l'on fait réagir le 4-méthyl-5-mercapto-méthyl-imidazole avec le chlorhydrate de la N-aziridino-N'-méthyl-N''-cyano-guanidine (tautomère : amino-méthyle et imino-nitrile, isomère géométrique qui détermine une basicité et permet donc la formation du chlorhydrate), dans un solvant comme par ex. de l'eau, un alcool, à une température pouvant par ex. aller de la température ordinaire à 100°C.

(7) Procédé selon la revendication 1 pour la préparation du composé de la formule :

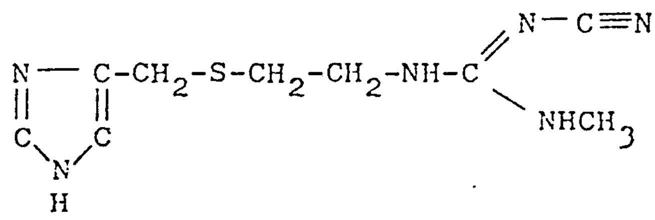

tautomère : amino-méthyle et imino-nitrile, isomère géométrique Syn($-NHCH_3$) - (différent de celui, Anti($-NHCH_3$) qui caractérise la cyano-guanidine de la même formule, déjà connue).

Caractérisé par ce que l'on fait réagir le chlorhydrate du 4-méthyl-5-chlorométhyl-imidazole avec la N-(2-mercapto-éthyl)-N'-méthyl-N''-cyano-guanidine (tautomère: amino-méthyle et imino-nitrile, isomère géométrique Syn($-NHCH_3$) (différent de l'isomère Anti($-NHCH_3$) du composé de même formule déjà connu) dans un solvant convenable comme par ex. de l'eau, en présence d'un agent alcalin comme par ex. KOH, NaOH, carbonate de potassium ou de sodium, dans une atmosphère inerte, par ex. d'azote ou d'argon, à une température allant par ex. de la température ordinaire à la température de 100°C.

(8) Procédé selon la revendication 1 et 3 pour la préparation d'une cyanamide mercurique :

Caractérisé par ce que l'on fait réagir de la cyanamide et de l'acétate mercurique dans de l'eau, isole le précipité blanc formé et le sèche convenablement.

(9) Procédé selon la revendication 1 et 3 pour la préparation d'une cyanamide mercurique :

Caractérisé par ce que l'on fait réagir une solution de cyanamide dans de l'eau avec une suspension de chlorure

mercurique dans de l'eau en présence de bicarbonate d'ammonium ou un bicarbonate alcalin et isole dans le précipité une cyanamide mercurique sous une forme moléculaire différente de celle de la préparation revendiquée dans la revendication 8.

0058286

Office européen des brevets

# RAPPORT DE RECHERCHE EUROPEENNE

Numéro de la demande

EP 81 81 0042

| DOCUMENTS CONSIDERES COMME PERTINENTS | | | CLASSEMENT DE LA DEMANDE (Int. Cl.³) |
|---|---|---|---|
| Categorie | Citation du document avec indication, en cas de besoin, des parties pertinentes | Revendication concernee | |
| | WO - A - 79/00466 (CRC)<br>* Pages 1-8 *<br>-- | 1,5,6 | C 07 D 233/64<br>203/12<br>C 07 C 129/08<br>149/437 |
| | EP - A - 0 020 173 (MERCK)<br>* Pages 41-44 *<br>-- | 1 | |
| P | WO - A - 81/00255 (SOC. DE RECH. ET DE SYNTH. ORGANIQUES)<br>* Pages 1-6 *<br>---- | 1,3,5,6 | |

DOMAINES TECHNIQUES RECHERCHES (Int. Cl.³)

C 07 C 233/64
203/12

CATEGORIE DES DOCUMENTS CITES

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

X Le présent rapport de recherche a été établi pour toutes les revendications

| Lieu de la recherche | Date d'achevement de la recherche | Examinateur |
|---|---|---|
| La Haye | 12-10-1981 | DE BUYSER |

OEB Form 1503.1 06.78